# EUROPÄISCHE PATENTANMELDUNG

(11) **EP 1 210 955 A2**
(43) Veröffentlichungstag der Anmeldung: **05.06.2002**
(21) Anmeldenummer: 01125751.6
(22) Anmeldetag: 29.10.2001
(51) Int. Cl.: A61M 1/06

(54) **Muttermilchpumpanordnung sowie Regeleinrichtung, Verteilventil und Pumpe hierfür**

(30) Priorität: 01.12.2000 DE 10059710
(71) Anmelder: Nüesch-Engineering, 5453 Remetschwil (CH)
(72) Erfinder: Nüesch, Hansueli, 5453 Rementschwil (CH)
(74) Vertreter: Révy von Belvárd, Peter

(57) **Zusammenfassung**

Eine Muttermilchpumpanordnung ist zum Anschluß zweier etwa trichterförmigen Brustkörper (19) über je eine Saugleitung (20) an eine Vakuumquelle (1') einer Pumpeneinheit (1) vorgesehen. An die Pumpeneinheit (1) ist eine Verteilventileinheit (2) mit Anschlüssen (18, 18') für die beiden Saugleitungen (20) einerseits und einem Verbindungskanal (13) mit der Vakuumquelle (1') der Pumpeneinheit (1) angeschlossen. Außerdem ist eine Regeleinrichtung (14) für den Saugdruck vorgesehen. Diese Regeleinrichtung (14) ist zwischen Pumpeneinheit (1) und Verteilventileinheit (14) geschaltet, und zwar zwischen Vakuumquelle (1') und Verbindungskanal (13). Bevorzugt weist die Regeleinrichtung eine Luftleitung (20.1, 31, 20.2) auf, deren wirksamer Querschnitt mittels einer Einstelleinrichtung (30-41) veränderbar ist. Zu diesem Zweck weist sie einen an eine Saugleitung (12, 20.1) der Pumpeneinheit (1) anschließbaren Eingangsanschluß (26) und einen an den Verbindungskanal (13) der Verteilventileinheit (14) anschließbaren Verbindungsanschluß (27) auf. Zwischen Eingangsleitung (26) und Verbindungsanschluß (27) bestimmt ein zwischen zwei Endstellungen bewegbares Stellglied (30) den wirksamen Querschnitt zwischen diesen beiden Leitungen (26, 27). Vorteilhaft sind Verteilventileinheit (14) und Pumpeneinheit (1) als baulich gesonderte und lösbare Einheiten ausgebildet, die über eine lösbare Verbindung (20.1, 20.2) aneinander anschließbar sind.

## Beschreibung

Die Erfindung bezieht sich auf eine Muttermilchpumpanordnung nach dem Oberbegriff des Anspruches 1. Ferner bezieht sich die Erfindung auf Einheiten für den Aufbau einer solchen Muttermilchpumpanordnung, nämlich auf eine Regeleinrichtung mit den Merkmalen des Oberbegriffes des Anspruches des Anspruches 3, auf ein Verteilventil nach Anspruch 9 sowie auf eine Pumpeinheit nach Anspruch 10.

Eine derartige Muttermilchpumpanordnung ist auf dem Markt und entspricht annähernd dem Zusammenbau einer Konstruktion nach der WO 98/30257 mit einem Regler etwa nach dem U.S.-Patent Nr. 6,042,560. Dabei bilden Pumpe, Verteilventil und Regler eine starre bauliche Einheit. Dies liegt nicht zuletzt daran, daß man bei dieser Version davon ausging, die Pumpeinheit mit der Reglereinheit als auf den Brustkörper und die Sammelflasche für die Milch aufsetzbare Baueinheit auszubilden. Dies geht allerdings bei einer Konstruktion nach der genannten WO nicht so einfach wie bei der Konstruktion nach dem U.S.-Patent Nr. 4,673,388, weil diese letztere nur für eine Brust ausgebildet war und daher des Verteilventils nicht bedurfte. In jedem Fall liegt der Regler unmittelbar vor dem Sammelgefäß für die Milch und war bei den bekannten Anordnungen von einem Falschluftregler, d.h. der Zufuhr von Falschluft über einen Nebenschluß, gebildet. Dies machte den Regler anfällig für etwaige aus dem Sammelraum überlaufende Milch, was teure Sicherheitsvorkehrungen erforderlich machte.

Anderseits sind auch Handpumpanordnungen, wie etwa nach dem U.S.-Patent Nr. 4,857,051, bekannt, bei welchen die elektrische Motorpumpe durch eine Handpumpe ersetzt ist und für eine Brust oder beide Brüste ausgebildet sein kann.

Es ist nun einerseits ohne teure Veränderung von Spritzgießwerkzeugen, ja der ganzen Konstruktion, praktisch nicht möglich, eine jeweils andere Anforderungen erfüllende ähnliche Muttermilchpumpanordnung aus Teilen der bekannten Anordnungen zu produzieren. Gerade für die Verhältnisse und Käuferwünsche in unterschiedlichen Ländern und für unterschiedliche Preiskategorien wäre es aber höchst wünschenswert, Varianten einer Muttermilchpumpanordnung auf kostengünstige Weise herstellen zu können. Anderseits ist der Regler nach der bekannten Anordnung relativ teuer in seiner Ausgestaltung.

Es ist daher Aufgabe der Erfindung, eine relativ kostengünstige Bauweise aufzuzeigen, die auch flexibel in ihrer Ausgestaltung sein kann. Erfindungsgemäß gelingt dies durch die kennzeichnenden Merkmale des Anspruches 1.

Durch die Anordnung des Reglers, welchen Typs er immer sein mag, zwischen Vakuumquelle und Verbindungskanal des Verteilerventils ist er durch das ihm nachfolgende Verteilerventil gegen überlaufende Milch geschützt. Anderseits ergibt sich dadurch - im Gegensatz zum Stand der Technik (vgl. auch die U.S.-Patente 4,964,851 und 5,810, 772) - eine bauliche Trennung von Pumpe und Verteilventil, die eine flexiblere, d.h. besser anpaßbare, Konstruktionsgestaltung insbesondere erlaubt. Vorteilhaft ist es aber auch, wenn der Regler von demjenigen Typ ist, wie er in Anspruch 2 definiert ist.

Eine besonders zweckmäßige Regeleinrichtung weist in diesem Zusammenhang die Merkmale des Anspruches 3 auf, wogegen ein erfindungsgemäßes Verteilventil die Charakteristiken des Anspruches 9 und eine erfindungsgemäße Pumpe diejenigen des Anspruches 10 besitzt.

Weitere Einzelheiten der Erfindung ergeben sich aus der nachfolgenden Beschreibung eines in der Zeichnung schematisch dargestellten, besonders bevorzugten Ausführungsbeispieles. Es zeigen:
- Fig. 1: die wesentlichen Teile einer erfindungsgemäße Muttermilchpumpanordnung in Perspektivansicht, wozu die
- Fig. 2 bis 4: vergrößerte Schnittbilder der Regeleinrichtung nach Fig. 1 veranschaulichen, wobei Fig. 2 ein Schnitt nach der Linie II-II der Fig. 1, Fig. 3 ein Schnitt nach der Linie III-III der Fig. 2 und Fig. 4 ein Schnitt nach der Linie IV-IV der Fig. 2 ist.

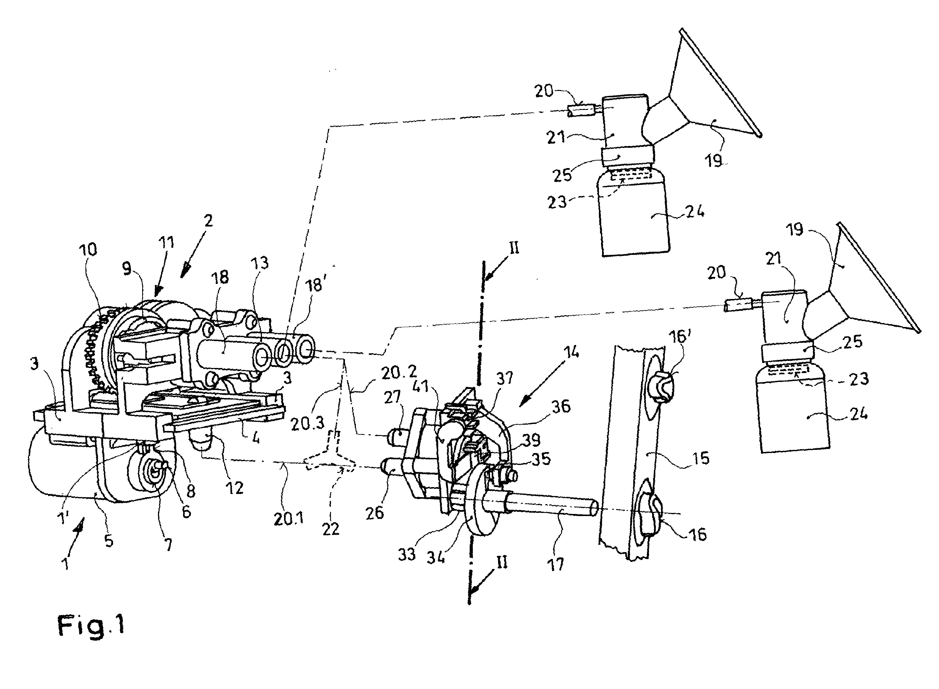

Gemäß Fig. 1 weist die erfindungsgemäße Muttermilchpumpanordnung eine Pumpeneinheit 1 und eine Verteilventileinheit 2 auf. Die Ventileinheit 2 ist fest mit einem Rahmen 3 mit Nuten verbunden, in welche eine hohle, einen Pumpenraum bildende Montageplatine 4 eingeschoben ist. An dieser Montageplatine 4 ist ein Elektromotor 5 befestigt, an dessen Motorwelle 6 über einen Exzenter 7 einen Pumpenstössel 8, vorzugsweise für den Antrieb einer an der Montageplatine 4 befestigte Membranpumpe 1' als Vakuumquelle zusammen mit dem Pumpenraum), gegebenenfalls aber für jede beliebige Art von Pumpe, wie eine Kolbenpumpe, zu einer auf- und abgehenden Pumpbewegung antreibt. Die Membrane der Pumpe ist in der Darstellung der Fig. 1 nicht sichtbar, entspricht aber beispielsweise der der EP-A-0 744 180, deren Inhalt hiermit durch Bezugnahme als geoffenbart gelten soll.

Somit ist die Pumpeinheit 1 durch Herausziehen aus dem Rahmen 3, z.B. auch zu Reparatur- oder Wartungszwecken, leicht auswechselbär. Falls gewünscht, kann eine Handpumpe, eine Kolbenpumpe oder eine andere Pumpe mit den restlichen Einheiten der erfindungsgemäßen Muttermilchpumpanordnung, wie der Ventileinheit 2, kombiniert werden. Es versteht sich aber, daß der Rahmen 3 auch Bestandteil eines Gerätegehäuses sein und eine weitere Nut zur Aufnahme einer weiteren Montageplatine für die Ventileinheit aufweisen kann.

Die Ventileinheit 2 kann an sich beliebiger Art, beispielsweise von der Art nach der schon genannten EP-A-0 744 180 oder der WO 98/30257, sein. Jedenfalls ist eine Ausführung mit hin- und hergehenden Ventilen gegenüber einer Lösung nach dem U.S.-Patent Nr. 4,964,851 mit einem sich drehenden Ventilkörper bevorzugt. Zur Betätigung mindestens eines Ventiles ist dann in bekannter Weise eine Nockenscheibe 9 vorgesehen, die ihren Antrieb entweder von einem eigenen Motor erhält, wie dies nach dem U.S.-Patent Nr. 6,045,529 vorgeschlagen worden ist, oder indem ein an der Motorwelle 6 befestigtes, nicht dargestelltes Motorritzel beim Einschieben der Pumpeinheit 1 mit einem Zahnrad 10 eines Untersetzungsgetriebes 11 für den Antrieb der Nockenscheibe 9 in Eingriff kommt.

Die Pumpeinheit 1 besitzt einen aus dem Pumpraum an der Montageplatine 4 herausführenden Verbindungskanalanschluß 12, auf den ein Verbindungsschlauch 20.1 steckbar ist. Dieser Verbindungsschlauch 20.1 steht mit einem weiteren Verbindungsschlauch 20.2 in Verbindung, der in die Mitte zwischen zwei Ventilkammern führt, und zwar über einen mittleren Verbindungskanalanschluß 13. Normalerweise, d.h. nach dem Stande der Technik, wären die Teile 12 und 13 in unmittelbarer Verbindung. Durch die Verwendung der Verbindungsschläuche 20.1 und 20.2 können die Einheiten 1 und 2 voneinander getrennt werden, um einen modularen Aufbau zu ermöglichen.

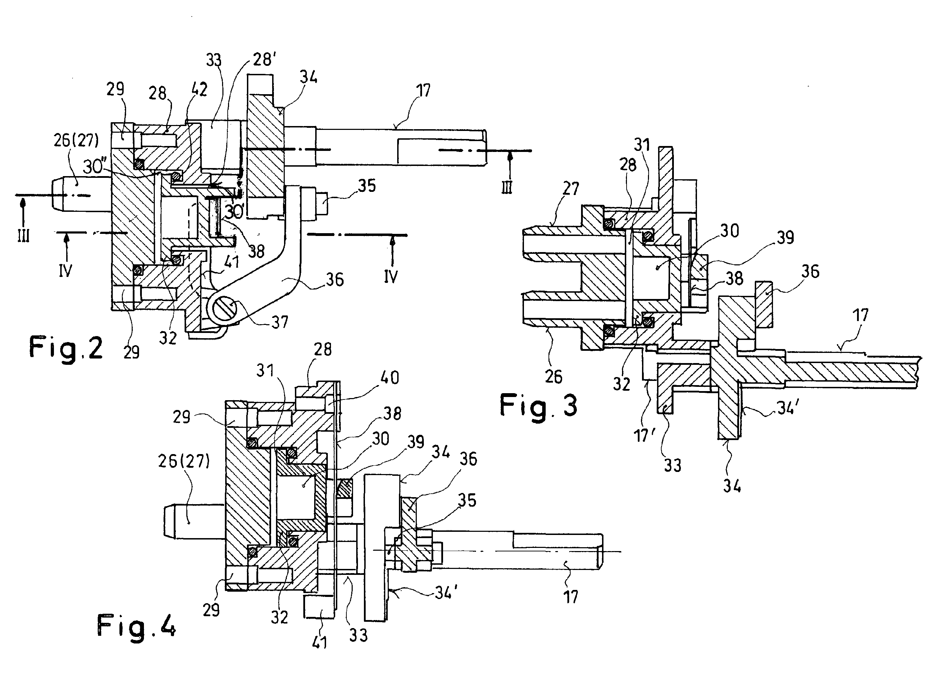

Überdies ist ersichtlich, daß natürlich auch durch Verwendung eines einzigen Verbindungsschlauches 20.1 bzw. 20.2 zwischen den Teilen 12 und 13 die herkömmliche Funktion, d.h. die Übertragung des Saugdruckes am Verbindungsanschluß 12 auf den Verbindungskanalanschluß 13, unmittelbar möglich wäre. Im vorliegenden, bevorzugten, Falle jedoch ist eine Reglereinheit 14 zwischengeschaltet, die später an Hand der Fig. 2 bis 4 im einzelnen erläutert wird. Diese Reglereinheit 14 ist vorzugsweise nahe einer Gehäusewand 15 angeordnet, um ein Justieren von außen mit Hilfe eines Einstellknopfes 16 zu ermöglichen, der an einer Einstellwelle 17 der Reglereinheit 14 befestigt ist. Obwohl diese Ausführung bevorzugt ist, sei erwähnt, daß alternativ die Reglereinheit 14 nur für eine Vorjustierung vor Auslieferung der erfindungsgemäßen Muttermilchpumpanordnung ausgebildet sein mag, so daß dann der Einstellknopf 16 wegfallen würde und sich die Einstellwelle 17 auch nicht durch die Gehäusewand 15 erstreckt, insbesondere kürzer als dargestellt und nur nach Öffnen des Gehäuses 15 durch einen Fachmann verstellbar ist. Natürlich entfällt der Einstellknopf 16 auch dann, wenn eine Reglereinheit 14 bei einer vereinfachten Ausführung der erfindungsgemäßen Muttermilchpumpanordnung gar nicht vorgesehen sein soll und die Schläuche 20.1, 20.2 - wie oben erwähnt - als einziger Kurzschlußschlauch ausgebildet sind. Ein weiterer Knopf 16' mag zum Einschalten des Motors 5 dienen. Es ist klar, daß die Erfindung nicht auf die Verwendung von Drehknöpfen beschränkt ist, sondern ebenso gut mit Schieber, Tastern oder jedem anderen Einstell- bzw. Schaltelement ausgerüstet werden könnte.

Die Reglereinheit 14 besitzt einen mit dem Verbindungskanalanschluß 12 der Pumpeneinheit 1 über den Verbindungsschlauch 20.1 in Verbindung stehenden Eingangsanschluß 26 und einen mit dem Verbindungskanalanschluß 13 der Ventileinheit 2 verbundenen Verbindungsanschluß 27. Im übrigen besitzt die Ventileinheit 2 in bekannter Weise je einen Anschluß 18, 18' bzw. eine entsprechende Auslaßbohrung, die über je einen Schlauch 20 zu einer Saugkammer 21 sowie zu einem Brustkörper 19 führt. An die Saugkammer 21 ist über ein Rückschlagventil 23 zum Abschluß eines Saugraumes 25 ein Milchsammelbehälter 24 angeschlossen.

Obwohl die Regeleinrichtung 14 an sich beliebig ausgebildet sein kann, ist die an Hand der Fig. 2 bis 4 gezeigte Ausführung bevorzugt. Dabei ist besonders aus Fig. 3 ersichtlich, wie die beiden Anschlüsse 26, 27 miteinander in Verbindung stehen. Beide Anschlüsse münden in einem mittels Schrauben oder Dübeln bei 29 (Fig. 2) angeschlossenen Reglergehäuse 28 gegenüber einem etwa topfförmigen Stellglied 30. Dieses Stellglied 30 ist innerhalb eines Zylinderraumes 31 zwischen einer linken und einer rechten Endlage (bezogen auf die Fig. 2 bis 4) bewegbar. Das Stellglied 30 besitzt eine Krempe bzw. einen Flansch 32, der nach Fig. 3 so bemessen ist, daß er die Mündungen der Anschlüsse 26, 27 im Reglergehäuse 28 mindestens teilweise zu überdecken und damit dies Anschlüsse 26, 27 mehr oder minder abzudecken vermag. Hier sei erwähnt, daß es zwar aus Symmetriegründen bevorzugt ist, wenn die Krempe 32 beide Anschlußeinmündungen abdeckt, doch würde es für die Funktion als regelbarer Strömungswiderstand (den das Stellglied 30 in der eben beschrieben, jedoch nicht bevorzugten Ausführung darstellt) ausreichen, wenn die Krempe bzw. der Flansch 32 einseitig nur eine der beiden Einmündungen der Anschlüsse 26, 27 abdeckt. In der dargestellten linken Stellung des Stellgliedes 30 deckt also die Krempe 32 die beiden Einmündungen vollständig ab und sperrt so jedwede Saugströmung ab. In der in Fig. 2 dargestellten rechten Endstellung hingegen, stehen beide Einmündungen miteinander in Verbindung. Jede Zwischenstellung des Stellgliedes verringert den Strömungsquerschnitt gegenüber der dargestellten rechten, d.i. voll geöffneten, Endstellung.

Wenn es also, wie oben erwähnt, erwünscht wäre, eine Reglereinstellung nur vor Auslieferung des Gerätes bzw. bei Nacheinstellungen durch einen Fachmann ausführen zu lassen, so könnte die rechte Endstellung des Stellgliedes 30 beispielsweise durch eine als Anschlag wirkende, an der rechten äußeren Fläche des topfförmigen Stellgliedes 30 Einstellschraube bestimmt werden. Bei der bevorzugten Ausführung (wie gezeigt) weist das Gehäuse 28 jedoch einen Lagerfortsatz 33 (vgl. Fig. 3) auf, in welchem ein Ende 17' der Einstellwelle 17 drehbar gelagert ist. Die Einstellwelle 17 trägt eine mit ihr drehfest verbundene Justiernocke 34, wie am besten aus den Fig. 1, 3 und 4 erkennbar ist.

An der Nockenfläche 34' der Justiernocke 34 liegt ein, besonders aus Fig. 1 ersichtlicher, Taststift oder Nockenfolger 35 an. Dieser Nockenfolger 35 sitzt gemäß den Fig. 1 und 2 am freien Ende eines Einstellhebels 36, dessen anderes Ende mit einer Welle 37 fest verbunden ist. An sich könnte damit ein mit der Welle 37 verbundener Endanschlag für die gewünschte Endlage des Stellgliedes 30 verbunden sein. Im vorliegenden bevorzugten Ausführungsbeispiel ist jedoch die Stellung des Stellgliedes statt eines starren Endanschlages eine durch die Kraft einer der Saugwirkung zwischen den beiden Einmündungen der Anschlüsse 26, 27 entgegenwirkenden Feder bestimmt. Diese Feder könnte verschieden, sei es als Druck- oder als Zugfeder, ausgebildet sein, doch ist man bei solchen Geräten räumlich meistens sehr beengt. Es hat sich daher als vorteilhaft erwiesen, wenn die Feder als Blattfeder 38 ausgebildet ist.

Bei der Darstellung nach den Fig. 2 bis 4 wirkt der im Zylinderaum 31 herrschende Sog im Sinne einer Bewegung des Stellgliedes 30 nach links. Das Stellglied 30 besitzt, wie besonders Fig. 3 deutlich zeigt, einen die Feder 38 übergreifenden Bügel 39, so daß das Stellglied 30 praktisch an der Blattfeder 38 elastisch aufgehängt ist. Die Blattfeder 38 ist an einem ihrer Enden, z.B. mittels einer Schraube 40 (Fig. 4) oder auf eine andere Weise mit dem Gehäuse 28 fest verbunden. Das andere, freie Ende der Blattfeder 38 stützt sich dagegen an einem Hebel 41 (Fig. 1, 2) derart ab, wie dies aus den Fig. 1 und 4 ersichtlich ist. Die Drehlage dieses Hebels 41 bestimmt also diejenige Kraft, die die Feder 38 auf den Bügel 39 - und damit auf das Stellglied 30 - ausübt. Die Drehlage des Hebels 41 wird aber dadurch bestimmt, daß er - wie die Fig. 1 und 2 veranschaulichen - an der Welle 37 befestigt ist, mit der auch der Einstellhebel 36 fest verbunden ist.

Wenn daher die Justiernocke 34 mittels der Welle 17 gedreht wird, so verschiebt sich der an ihr anliegende Nockenfolgerstift 35 entsprechend der jeweiligen axialen Stärke des Nockenkörpers. Der Nockenfolger 35 liegt aber an der Axialkurve der Nockenfläche 34' unter der Kraft der Blattfeder 38 an, die hier also in vorteilhafter Weise eine doppelte Funktion, nämlich die der Belastung des Stellgliedes 30 nach rechts und des Anpressens des Nockenfolgers 35 an die Nockenfläche 34', die sie dadurch erfüllt, daß die Blattfeder 38 ein Drehmoment auf den mit der Welle 37 verbundenen Hebel 41 und damit auch auf den den Nockenfolger 35 tragenden Einstellhebel 36 ausübt.

Bei der obigen Beschreibung an Hand der Fig. 2 bis 4 wurde das Stellglied 30 so beschrieben, daß dessen Krempe 32 zylindrisch ist und den gesamten Durchmesser des Zylinderraumes 31 dichtend ausfüllt. Der dargestellte Regler ist aber gewünschtenfalls (und bevorzugt) leicht auch auf einen Falschluftregler abzuändern, wenn das Stellglied 30 derart ausgebildet wird, daß es im rechten, topfförmigen Teil gegenüber der ihn aufnehmenden Gehäusebohrung 28' (Fig. 2) mindestens über einen Teil seines Umfanges in der aus Fig. 2 ersichtlichen Weise einen Spalt freiläßt und so eine Luftnebenschlußleitung 30' bildet.

Ebenso liegt dann die Krempe 32 nicht dichtend an der Zylinderwand des Raumes 31, sondern läßt einen Spalt 30" frei, der mit dem Spalt 30' in Verbindung zu treten vermag. In der aus Fig. 2 ersichtlichen rechten Endstellung des Stellgliedes 30 liegt aber die Krempe 32 an einem Dichtungsring 42 an, so daß die Luftnebenschlußleitung über die Bohrung 30' abgeschlossen ist. Erst wenn sich das Stellglied 30 aus der gezeigten Stellung nach links bewegt, wird diese Luftnebenschlußleitung mehr oder minder freigegeben.

Bei einer solchen Konstruktion könnte an Stelle eines zylindrischen Raumes 31 ein sich nach links zu erweiternder, leicht konischer Raum 31 vorgesehen werden. Die eben an Hand der Fig. 2 geschilderte Ausbildung erleichtert selbstverständlich eine flexible Anpassung der erfindungsgemäßen Muttermilchpumpanordnung an unterschiedliche Anforderungen.

Beispielsweise könnte ein Falschluftregler 14 der oben beschriebenen Bauweise verwendet werden, bei dem aber der Anschluss 27 (Fig. 1) weggelassen oder zugestopft ist. Statt dessen wirkt die Beeinflußung des Saugdruckes durch den Zustrom von Falschluft unmittelbar auf ein lediglich strichliert dargestelltes Y-Stück 22 ein, das eine Parallelschaltung von Regler 14 und Ventileinheit 2 bewirkt, d.h. die Falschluft strömt zu einem Saugpfad, der von der Leitung 20.1 einerseits und einer direkt zum Anschluß 13 führenden Leitung 20.3 anderseits gebildet wird. Dies würde den Regler 14 selbst dann gegen das Eindringen von Milch schützen, wenn eine solche bis über die Ventileinheit 2 hinaus in Richtung zur Pumpeinheit 1 gelangen sollte, denn der Falschluftstrom ist ja entgegengesetzt. Dies kann deshalb von Vorteil sein, weil die Reglereinheiten normalerweise anfällig für Verschmutzungen sind, und diese Anordnung einen zusätzlichen Schutz gewährleisten kann. Anderseits geht dies um den zusätzlichen Preis des Y-Stückes 22 und der zusätzlichen Schlauchverbindung zum Anschluß 26, weshalb die erstgenannte Variante mit dem Schlauch 20.2 dort bevorzugt sein wird, wo die Verschmutzungsgefahr durch andere konstruktive Maßnahmen ausgeschaltet werden kann.

Im Rahmen der Erfindung sind zahlreiche Varianten möglich; beispielsweise könnte der Zylinderraum 31 nach rechts zu erweitert werden, wobei der an den Leitungen 20.1, 20.2 liegende Sog auf die rechte Seite des Stellgliedes 30 gelenkt wird. In diesem Falle könnte die Feder 38 so ausgebildet werden, daß sie das Stellglied 30 gegen die die Leitungen 20.1 bzw. 20.2 verschließende bzw. den Strömungswiderstand erhöhende Endlage hin belastet. Es ist aber ersichtlich, daß die gezeigte bevorzugte Lösung einfacher ist.

An Stelle einer einzigen Blattfeder 38 könnten auch deren mehrere, etwa als Blattfederpaket, vorgesehen sein, beispielsweise um der Feder eine gewünschte Charakteristik zu verleihen.

Ferner könnte zum Verstellen der Endstellung des Stellgliedes 30 bzw. der Kraft der Feder 38 an Stelle einer Axialkurve, wie dargestellt, auch eine Radialkurve verwendet werden, obwohl die gezeigte Axialkurve 34' insofern günstiger ist, als sie einen geringeren Platzbedarf aufweist.

Es wurde oben beschrieben, wie eine lösbare Verbindung zwischen den einzelnen Einheiten 1, 2 bzw. 14 mittels Schläuchen 20.1, 20.2 bewerkstelligt wird. Natürlich könnte eine lösbare Verbindung auch mittels fester, jeweils über Kupplungen oder Flanschverbindungen aneinander anschließbarer Leitungselemente erzielt werden.

Natürlich könnte auch das Stellglied 30 als einstellbarer Strömungswiderstand auf die unterschiedlichste Weise ausgebildet sein. Beispielsweise könnte es sich um einen Schieber mit Düsen unterschiedlichen Querschnitts handeln, von denen jweils eine in den Strömungsweg geschaltet wird. Eine andere Alternative könnte von einer gegenüber und in einer Düsenöffnung verschiebbaren Ventilnadel gebildet sein. Aus dem Ventilbau sind ferner noch die verschiedensten Alternativen bekannt.

## Patentansprüche

1. Muttermilchpumpanordnung zum Anschluß zweier etwa trichterförmigen Brustkörper (19) über je eine Saugleitung (20) an eine Vakuumquelle (1') einer Pumpeneinheit (1), an die eine Verteilventileinheit (2) mit Anschlüssen (18, 18') für die beiden Saugleitungen (20) einerseits und einem Verbindungskanal (13) mit der Vakuumquelle der Pumpeneinheit (1) angeschlossen ist, sowie mit einer Regeleinrichtung (14) für den Saugdruck, **dadurch gekennzeichnet, daß** die Regeleinrichtung (14 über eine lösbare Verbindung, z.B. über eine Schlauchverbindung (20.1, 20.2), mit der Anordnung verbunden ist und/oder zwischen Pumpeneinheit (1) und Verteilventileinheit (14), und zwar zwischen Vakuumquelle (1') und Verbindungskanal (13) geschaltet ist.

2. Anordnung nach Anspruch, **dadurch gekennzeichnet, daß** die Regeleinrichtung (14) als Saugdruck beeinflußendes Glied zwischen Vakuumquelle (1') und Verbindungskanal (13) geschaltet ist und daß vorzugsweise der Saugwiderstand über ein von außen zugängliches Verstellelement (16) einstellbar ist.

3. Regeleinrichtung für eine Muttermilchpumpanordnung nach Anspruch 1 oder 2, mit einer Luftleitung (20.1, 31, 20.2; 20.3), deren wirksamer Luftdurchsatz bzw. Saugdruck mittels einer Einstelleinrichtung (30-41) veränderbar ist, **dadurch gekennzeichnet, daß** sie einen an eine Saugleitung (12, 20.1) der Pumpeneinheit (1) anschließbaren Eingangsanschluß (26) aufweist, der mit dem Verbindungskanal (12) zur Vakuumquelle (1') verbunden ist, und daß vorzugsweise wenigstens eines der folgenden Merkmale vorgesehen ist:
a) die Regeleinrichtung weist einen an den Verbindungskanal (13) der Verteilventileinheit (14) anschließbaren Verbindungsanschluß (27) auf;
b) die Regeleinrichtung ist als Falschluftregler ausgebildet und/oder über ein Y-Stück (22) sowohl mit der Saugleitung (20.1) der Pumpeinheit (1) als auch mit dem Verbindungskanal (13) der Verteilventileinheit (14) verbunden.

4. Regeleinrichtung nach Anspruch 3, **dadurch gekennzeichnet, daß** die Regeleinrichtung (14) zwischen Eingangsleitung (26) und Verbindungsanschluß (27) ein den wirksamen Luftdurchsatz bzw. Saugdruck zwischen diesen beiden Leitungen (26, 27) bestimmendes, zwischen zwei Endstellungen bewegbares Stellglied (30) besitzt.

5. Regeleinrichtung nach Anspruch 3 oder 4, **dadurch gekennzeichnet, daß** die Regeleinrichtung (14) zwischen Eingangsleitung (26) und Verbindungsanschluß (27) ein aus einer eine Luftnebenschlußöffnung abdeckende in mindestens eine diese Öffnung freigebende Lage bewegbares Stellglied (30) besitzt.

6. Regeleinrichtung nach einem der Ansprüche 3 bis 5, **dadurch gekennzeichnet, daß** das Stellglied (30) von einer den Luftdruck bestimmenden Feder (38) in Richtung auf eine seiner Endstellungen vorgespannt ist, wobei vorzugsweise
a) das Stellglied (30) in Richtung auf die eine maximalen Durchströmquerschnitt ergebende Endstellung vorgespannt ist oder
b) das Stellglied (30) in Richtung auf das Schließen der Luftnebenschlußöffnung vorgespannt ist.

7. Regeleinrichtung nach Anspruch 6, **dadurch gekennzeichnet, daß** die Kraft der Feder (38) mittels einer Einstelleinrichtung (17, 34-37, 41) verstellbar ist, welche gegebenenfalls eine Einstellkurve (34) aufweist, deren Kurvenform über mindestens einen Hebel (36 bzw. 41) auf das Stellglied (30) bzw. einen damit verbundenen Teil, vorzugsweise die Feder und insbesondere die Blattfeder (38), übertragbar ist, und daß bevorzugt die Kurve (34') über das freie Ende eines ersten Hebel abtastbar ist und über einen weiteren Hebel auf das Stellglied bzw. einen damit verbundenen Teil, vorzugsweise die Feder und insbesondere die Blattfeder, übertragbar ist und/oder zweckmäßig die Kurve (34') als Axialkurve ausgebildet ist.

8. Regeleinrichtung nach Anspruch 6 oder 7, **dadurch gekennzeichnet, daß** die Feder mindestens eine Blattfeder (38) aufweist, und daß vorzugsweise das Stellglied (30) in einem mittleren Bereich an der Blattfeder (38) angreift, wogegen in ihrem Endbereich eine Einstelleinrichtung (41) für die Federkraft angreift.

9. Verteilventil für eine Muttermilchpumpanordnung nach einem der vorhergehenden Ansprüche, welches mit dem Sauganschluß (12) der Pumpe (1) in Verbindung steht, **dadurch gekennzeichnet, daß** es eine von der Pumpe (1) baulich gesonderte Einheit (14) bildet und an den Sauganschluß (12) der Pumpe (1) über eine lösbare Verbindung (20.1, 20.2) anschließbar ist, insbesondere für die lösbare Verbindung ein an den jeweiligen Anschluß (12, 13) ansteckbarer Schlauch (20.1, 20.2) vorgesehen ist.

10. Pumpeinheit für eine Muttermilchpumpanordnung nach einem der vorherigen Ansprüche, welche mit dem Verteilventil (14) in Verbindung steht, **dadurch gekennzeichnet, daß** sie eine vom Verteilventil (14) baulich gesonderte und lösbare Einheit (1) bildet und an den Verbindungskanal (13) desselben (14) über eine lösbare Verbindung (20.1, 20.2) anschließbar ist, insbesondere für die lösbare Verbindung ein an den jeweiligen Anschluß (12, 13) ansteckbarer Schlauch (20.1, 20.2 bzw. 20.3) vorgesehen ist.
